# EUROPÄISCHE PATENTSCHRIFT

(11) **EP 1 286 708 B1**
(45) Veröffentlichungstag und Bekanntmachung des Hinweises auf die Patenterteilung: **07.04.2004**
(21) Anmeldenummer: 01943149.3
(22) Anmeldetag: 28.05.2001
(51) Int. Cl.: A61L 27/38, A61L 27/50

(54) **VERFAHREN ZUR HERSTELLUNG EINES BIOARTIFIZIELLEN TRANSPLANTATES**
METHOD FOR PRODUCING A BIO-ARTIFICIAL TRANSPLANT
PROCEDE DE PRODUCTION D'UN TRANSPLANT BIO-ARTIFICIEL

(30) Priorität: 29.05.2000 DE 10026482
(43) Veröffentlichungstag der Anmeldung: 05.03.2003
(73) Patentinhaber: Bader, Augustinus, 31275 Lehrte-Immensen (DE)
(72) Erfinder: Bader, Augustinus, 31275 Lehrte-Immensen (DE)
(74) Vertreter: Müller, Volkmar
(86) Internationale Anmeldenummer: PCT/DE2001/001986
(87) Internationale Veröffentlichungsnummer: WO 2001/091820

(56) Entgegenhaltungen:
- WO-A-94/03584
- WO-A-99/62424
- DE-A- 19 828 726

## Beschreibung

Die Erfindung betrifft ein Verfahren zur Herstellung eines bioartifiziellen Transplantates aus einem für die Transplantation vorgesehenen biologischen Gewebe und darauf aufgebrachten empfängervertäglichen Zellen.

Allgemein betrifft die Erfindung ein Verfahren zur kontrollierten Züchtung von biologischem Gewebe.

In der Transplantationsmedizin besteht ein großes Bedürfnis nach geeigneten, in möglichst geringem Maße adverse Reaktionen des Transplantat-Empfängers auslösenden Transplantaten. Nur in bestimmten Fällen ist es möglich, das Transplantat aus dem Körper des Empfängers selbst zu entnehmen und zu verpflanzen. Aus immunologischer Sicht sind diese Transplantationen am unbedenklichsten, bei bestimmten Gefäßen oder Organen sowie bei größeren zu ersetzenden Hautbereichen besteht diese Möglichkeit jedoch nicht. Für bestimmte Organe kommen heute praktisch nur allogene Transplantate fremder Spender oder - vielfach im orthopädischen Bereich - synthetische Implantate aus Kunststoffen, Metallen, Keramik usw. bzw. verschiedenen Verbundstoffen in Betracht. Bei der Verwendung allogener Materialien, wie z.B. Spenderorganen ist eine ständige für den Organismus des Empfängers belastende Immunsuppression nötig. Dennoch kommt es häufig zu Abstoßungsreaktionen als ernsten Komplikationen. Auch künstliche Materialien können zu Abstoßungsreaktionen und entzündlichen Prozessen führen, die das Operationsergebnis zunichte machen.

Aus verschiedenen Gründen wird heute oft versucht, xenogenes Material (tierischen Urprungs) zu verwenden. Vorteilhaft ist hierbei vor allem die bessere Verfügbarkeit dieses Materials gegenüber allogenen (Spender-)Materialien. Auch ist ein solches "biologisches Material" flexibler als ein künstliches Material und paßt sich an manchen Stellen dem Empfängerkörper besser an. Das xenogene Transplantationsmaterial ist jedoch deshalb problematisch, da es stark antigen ist.

Es wird daher seit längerer Zeit versucht, xenogene Transplantationsmaterialien - speziell verschiedene für eine Transplantation vorgesehene Gewebe - empfängerverträglich zu machen. Hierzu wird in der Regel versucht, innerhalb oder auf der strukurgebenden Bindegewebsmatrix des xenogenen Transplantats eingebettete native Zellen abzutöten oder zu entfernen sowie Fremdproteine und andere Fremdsubstanzen auszuspülen. Die strukturgebende Matrix aus interstitiellem Bindegewebe kann als eine immunologisch weitgehend neutrale Matrix angesehen werden.

Chemisch behandelte Transplantate tierischer Herkunft werden beispielsweise für den Herzklappenersatz bei Menschen verwendet. Das tierische Material wird dabei im allgemeinen mit Glutaraldehyd behandelt, um die Strukturproteine zu stabilisieren und eine antigene Reaktion zu verhindern. Das mit Glutaraldehyd behandelte Gewebe unterliegt jedoch einer stetigen Verhärtung und fortschreitenden Calzifizierung nach der Transplantation. Diese Transplantate müssen daher alle paar Jahre ersetzt werden.

Ersatzweise wurde auch bereits versucht, eine azellularisierte und hierdurch "neutralisierte" freiliegende Collagenmatrix zu transplantieren, was jedoch, wie sich zeigte, ebenfalls Probleme mit sich bringt. Die azellularisierte Collagenmatrix ist durch den Azellularisierungsvorgang stark aufgelockert und mechanisch instabil. Ein schwerwiegender Nachteil der Destabilisierung ist die Gefahr von Initialversagen nach Implantation im Körper durch Ruptur. Dies trat bei Tiermodellen in 20 % der Fälle auf (azellularisierte Schweineherzklappen, rebesiedelt mit autologen Zellen im Niederdruckkreislauf, Pulmonalposition).

Zusätzlich wird eine freiliegende Collagenmatrix im Körper durch Collagenasen leicht angegriffen, so dass es zu Schädigungen kommen kann, bevor eine Re-Besiedlung im Körper stattfinden könnte.

Es ist daher ebenfalls versucht worden für eine Transplantation vorgesehene azellularisierte biologische Gewebe vor der Transplantation bereits mit autologen oder allogenen Zellen wiederzubesiedeln. In der DE 19828726 A1 wird beispielsweise ein Verfahren zur Herstellung eines bioartifiziellen Transplantats beschrieben, bei dem zunächst native Zellen auf dem interstitiellen Bindegewebe des Transplantats abgetötet und dann entfernt werden. Die Matrix wird danach mit für den Empfänger verträglichen, vorzugsweise autologen Zellen neu besiedelt, so daß ein empfängerspezifisches Biotransplantat erhalten wird.

Hierbei ist bereits sehr vorteilhaft, dass antigene Komponenten weitgehend entfernt oder abgeschirmt sind. Das auf diese Weise erhaltene bioartifizielle Transplantat besitzt jedoch noch nicht die geforderten "natürlichen" Eigenschaften. Das Anwachsen der Zellen auf der azellularisierten aufgelockerten Matrix ist erschwert. Durch eine selbst geringe, Veränderung der Matrixstruktur wird auch kein vollkommen natürlicher Wiederaufbau der Zellen erhalten. Es bestehen auch noch erhebliche Probleme, das erforderliche Anwachsen verschieden differenzierter Zellen an den jeweils erforderlichen Orten zu steuern.

Aus der US PS 5192312 (Orton) ist bereits ein Besiedlungsverfahren bekannt, bei welchem eine implantierbare menschliche Herzklappe mit Fibroblasten-Wachstumsfaktor behandelt und danach mit einer solchen Menge Fibroblasten besiedelt wird, von der vermutet wird, das Implantat nicht-immunogen zu machen. Die Vorbereitung mit Wachstumsfaktoren behindert dieses Ziel, da eine primäre Maskierung mit exogenem Wachstumsfaktor zu funktionellen Veränderungen der aufzubringenden Zellen und einer exogen induzierten Verschiebung in einen proliferatorischen Phänotypus führen kann. Die exogene Zugabe von Wachstumsfaktoren führt zu internen Konkurrenzmechanismen in der Signalkommunkation, die letztendlich dazu führen, dass zwar viele Zellen entstehen, diese jedoch durch den Wachstumsfaktor nicht in der Lage sind, notwendige Remodellingprozesse einzuleiten. Die rasche Fertigstellung eines autologisierten Implantats hinsichtlich der Stützstrukturen wird hierdurch bereits in vitro verhindert. Eine Nachwirkung in vivo ist wahrscheinlich, da transformierte Zellen entstehen können. Dies hat weitere erhebliche Konsequenzen nach der Implantation in vivo, da allogene und xenogene Matrices immunogene Restwirkungen haben, die zu inflammatorischen Reaktionen und der Ausbildung von Verkalkungsfoci und damit zu langfristigen Transplantatversagen führen.

Der Erfindung liegt daher das Problem zugrunde, dass ein für eine Transplantation ausgewähltes für den Transplantations-Empfänger fremdes biologisches Gewebe, insbesondere ein allogenes oder xenogenes Material, zu einem empfängervertäglichen immunologisch aktzeptablen bioartifiziellen Transplantat umgeformt werden soll.

Weiterhin soll ein Verfahren geschaffen werden, das mechanisch stabilere, naturähnlichere Transplantate erzeugt. Der Umbildungsprozess soll bei gleichzeitiger Stimulierung und Beschleunigung des natürlichen Umbaus möglichst kontrolliert ablaufen.

Zur Lösung dieses Problems ist erfindungsgemäß vorgesehen, dass bei einem Verfahren zur Herstellung eines bioartifiziellen Transplantates aus einem für die Transplantation vorgesehenen biologischen Gewebe und darauf aufgebrachten empfängerverträglichen Zellen,
einem für die Transplantation vorgesehenen autologen, alloge nen oder xenogenen, in nativer Form (nicht azellularisiert) vorliegenden und nicht mit exogenen Wachstumsfaktoren vorbehandelten Gewebe in einem konditionierenden Medium empfängerverträgliche Zellen zugeführt werden, die wenigstens ausgewählte zu einem Remodelling der Trägerstrukturen befähigte Zellen umfassen,
die Behandlung des Transplantats fortgesetzt wird bis eine weitgehende Umbildung des ursprünglichen nativen Gewebes in ein im wesentlichen die zugeführten empfängerspezifischen Zellen aufweisendes Gewebe erzielt worden ist.

Unter "zu einem Remodelling der Trägerstrukturen befähigten Zellen" werden solche verstanden, die dazu beitragen, neue Gewebematrix zu sezernieren und vorzugsweise auch abgestorbene Zellen abzuräumen. Zu diesem Typus gehören je nach Gewebeart verschiedene Zellen, z.B. Fibroblasten bzw. Bindegewebszellen sowie deren Vorlauferzellen aus bevorzugt autologen Stammzellen.

Zu den zum Remodelling befähigten Zellen gehören im cardiovaskulären Bereich z.B. die glatten Muskelzellen. Allgemein zählen beispielsweise auch Makrophagen hinzu.

Die genannten Zellen fördern und Beschleunigen eine Gewebeumbildung; in der Regel ermöglichen sie die Umbildung dadurch erst, da anderenfalls andere Prozesse (Calzifizierung, Abstoßung) die Geweberegeneration oder Gewebeumbildung zeitlich "überholen" und auf diese Weise verhindern würden.

Die Anregung zur Gewebeumbildung kann auch durch einen gezielten Entzündungsreiz erfolgen, der Vorgänge zur Gewebeheilung stimuliert. Bei den zum Remodelling befähigten Zellen kann es sich daher auch oder teilweise um Zellen handeln, die Entzündungsmediatoren freisetzen können. Die Gewebeheilung bringt dann eine beschleunigte Gewebeumbildung mit sich. Die Entzündungsmediatoren können jedoch auch bei Verwendung anderer zum Remodelling befähigter Zellen zusätzlich zugeführt werden. Dies geschieht dann insbesondere zeitlich begrenzt, so dass ein kontrollierbarer heilungsstimulierender Entzündungsprozess eingeleitet wird.

Zu den Stammzellen werden gerechnet: Knochenmarkszellen, aus Fettgewebe stammende (mesenchymale) Zellen, gewebespezifische Stammzellen, Stammzellen aus peripherem Blut, organspezifische Stammzellen sowie Zellen nach autologem Kerntransfer, beispielsweise körpereigene Muskelzellkerne in Fibroblasten (mit erfolgender Transdifferenzierung).

Der Erfindung liegt das grundlegend neue Konzept einer kontrollierten Geweberegeneration in vitro zugrunde. Anders als bei den bisher angewendeten Verfahren werden die nativen Zellen des zur Transplantation vorgesehenen Gewebes weder wie bisher üblich entfernt noch notwendigerweise künstlich abgetötet. Das Gewebe wird vielmehr in einer geeigneten Vorrichtung, die ein üblicher Besiedlungsreaktor sein kann, einer künstlichen "Wundheilung" unterzogen; dabei wird bereits primär durch gewebeeigene Mediatoren, ein Stimulationsreiz auf neu anwachsenden Zellen ausgeübt.

Unter einem Gewebe in nativem Zustand (nicht azellulariziert) wird ein Gewebe wie präpariert, d.h. dem xenogenen oder allogenen Spender entnommen, verstanden. Die in dem Gewebe vorhandenen Zellen, die sie ab Präparation oder Entnahme in einem Zustand des Absterbens befinden, werden entsprechend den Prinzipien dieser Erfindung nicht vor der Weiterbehandlung in gesonderten Verfahrensschritten entfernt.

Durch die Zuführung von für den Empfänger verträglichen und zu der Gewebeart passenden Zellen wird das ursprünglich fremde Transplantat während der Behandlungsphase allmählich zu einem für den Empfänger völlig immunverträglichen bioartifiziellen Transplantat umgebildet.

Der Erfindung liegt die Erkenntnis zugrunde, dass das Entfernen oder auch aggressive Abtöten der ursprünglichen nativen Zellen eines fremden allogenen oder xenogenen Gewebes die Wiederbesiedlung erschwert hat, und zwar insbesondere auch deshalb, weil ein von diesen Zellen ausgehender Reiz für die natürliche in jedem Körper vor sich gehende ständige Zellerneuerung verloren geht. Insbesondere wurden dadurch wichtige Schüsselfaktoren für den letztendlich erforderlichen Matrixumbau sowie für eine effiziente Matrixneusynthese entfernt.

Zusätzlich bewirkte die Azellularisierung eine erhebliche Destabilisierung, die hinsichtlich einer klinisch erforderlichen guten Initialstabilität für Implantationszwecke jedoch dringend erforderlich ist. Die Erfindung löst diese Probleme.

Solange das fremde, noch mit nativen Zellen besiedelte xenogene oder allogene für die Transplantation vorgesehene Gewebe in seinem nativen Zustand belassen wird, werden bei Kultur bzw. Inkubation des Gewebes in einer beispielsweise aus Nährmedium bestehenden konditionierenden Umgebung durch Zellen des umzubildenden Gewebes zelluläre Mediatoren freigesetzt, die einen natürlichen Umbau begünstigen (endogener Stimulus). Die Mediatoren verteilen sich auf bestimmten Wegen innerhalb des Gewebes und wandern zum geringen Teil in das konditionierende Medium aus. Werden nun während der Kultur des für die Transplantation vorgesehen Gewebes, das noch mit seinen nativen Zellen ausgestattet ist, in dem konditionierenden Medium schubweise oder laufend neue empfängerverträgliche Zellen zugeführt, werden diese in den Umbildungsprozeß miteinbezogen und ersetzen mit der Zeit die nativen Zellen, die bei Austausch des konditionierenden Mediums allmählich mit abgeführt werden. Dabei ist es wesentlich, dass die empfängerverträglichen Zellen ausgewählte zu einem Remodelling der Trägerstrukturen befähigte Zellen, z.B. Bindegewebszellen oder Fibroblasten, zumindest mit umfassen. Daneben können weitere empfängerverträgliche Zellen vorhanden sein. Die jeweils zu verwendenen Zellen kann der Fachmann entsprechend den oben gemachten Angaben und Erläuterungen passend zu dem umzubildenden Gewebetyp auswählen.

Grundsätzlich ist es nämlich bekannt, dass natürliche - auch nicht-azellularisierte, beispielsweise nicht-denaturierte allogene Transplantate an ihrer Oberfläche mit Endothelzellen besiedelt werden können. Dies geschieht auch nach der Transplantation spontan in vivo, wenn sich im Körper Endothelzellen auf ein allogenes oder xenogenes Transplantat aufsiedeln. Eine solche Endothelialisierung führt jedoch nicht zu einer wirklichen Umbildung bzw. einem "Remodelling" des Transplantatgewebes. Durch immunologische Prozesse setzen recht bald an dem fremden (und fremd bleibenden) Gewebe von einzelnen Focuspunkten (Verkalkungsfoci) ausgehende Verkalkungsprozesse ein. Das transplantierte Gewebe oder Organ wird dabei im Laufe der Zeit immer stärker geschädigt und schließlich funktionsuntüchtig.

Tierversuche zeigen, dass beispielsweise Herzklappen bereits innerhalb von 24 bis 48 Stunden spontan endothelialisieren. Dabei wird jedoch das Gewebe nicht umgebaut sondern verdichtet. Die Endothelzellen verbleiben physiologischerweise an der Oberfläche. Wie L. Maxwell, J.B. Gavin, B.G. Barratt-Boyes, in "Differences between heart valve allografts and xenografts in the incidence and initiation of dystrophic calcification", Pathology (1989, 21, 5-10) untersucht haben, führt die Anwesenheit restlicher Spenderzellen zu Kalzifizierungsnestern, die letztendlich ein Klappenversagen bewirken.

Die Abstoßung und Verkalkung des transplantierten Gewebes bzw. Organs kann durch das erfindungsgemäße Verfahren vermieden werden, da bereits vor der Transplantation ein Remodelling in vitro durchgeführt wird, bei dem das für die Transplantation vorgesehene Gewebe weitgehend umgebaut wird.

Hierfür ist es notwendig, dass die innerhalb des Verfahrens verwendeten empfängerspezifischen Zellen zu einem Remodelling befähigte Zellen mit umfassen. Zu diesen gehören u.a. die Fibroblasten, die durch Umgebungsreize dazu veranlasst werden können, neue Matrix zu sezernieren und die Entfernung alter Zellen zu fördern. Andere Zelltypen können zusätzlich - gemischt mit den Fibroblasten, in verschiedenen Schichten oder Bereichen des Gewebes - verwendet werden.

Vorzugsweise werden die empfängerverträglichen Zellen einmalig zu Beginn der Kultur, d.h. der Behandlung des Transplantats, mehrfach in Intervallen oder kontinuierlich innerhalb des Mediums zugeführt.

Dabei können die empfängerverträglichen Zellen auf das umzubildende native Gewebe aufgetropft oder aufgestrichen, oder kontinuierlich oder schubweise mit dem konditionierenden Medium zugeführt werden.

Die dem umzubildenden Transplantat zuzuführenden empfängerversträglichen bzw. empfängerspezifischen Zellen können in bestimmten Ausführungsformen vermischt mit einem biologisch verträglichen Kleber, der insbesondere Fibrin, Kollagen Kleberproteine aus Muscheln oder synthetische Kleberproteine enthalten kann, oder in einer Kulturmedium-Suspension aufgegeben werden.

Die Behandlung des Transplantats kann unter mehrmaligem Austausch oder bei kontinuierlichem Durchlauf des Mediums, das ein übliches Kulturmedium sein kann, durchgeführt werden.

Die Umbildung wird vorzugsweise mechanisch dadurch unterstützt, dass das das Gewebe umspülende Kulturmedium bewegt wird und ein Flüssigkeitsstrom für das Antransportieren neuer empfängerverträglicher Zellen vorhanden ist, der bei der Umbildung abgestoßene/ersetzte Zellen mit fortspült. Das Gewebe kann zwischendurch - einmalig oder in Intervallen - gespült werden, wodurch ein mechanischer Reiz ausgeübt wird, der die Ablösung zu ersetzender Zellen begünstigt.

Wesentlich für die Erfindung ist daher, dass die Behandlung des Transplantates mit den empfängerverträglichen Zellen in dem konditionierenden Medium unter mehrmaligem Austausch oder bei kontinuierlichem Durchfluß des Mediums so lange fortgesetzt wird, bis ein weitgehender Umbau des ursprünglichen nativen Gewebes in ein solches Gewebe erfolgt ist, das im wesentlichen nur die zur Besiedlung verwendeten empfängerspezifischen Zellen aufweist.

Die Behandlung des nativen Gewebes in dem konditionierenden Medium mit empfängerverträglichen Zellen, wobei das konditionierende Medium entweder kontinuierlich umgewälzt oder mehrmals ausgetauscht wird, entspricht einer an sich bekannten Besiedlung einer unterliegenden Matrix mit Zellen, wie sie im Stande der Technik bekannt ist und in verschiedenen Varianten durchgeführt werden kann.

Als konditionierendes Medium kann ein übliches Zellkultur-Nährmedium verwendet werden, das ggf. mit verschiedenen Zusätzen versehen sein kann. Hierfür geeignete Nährmedien sind dem Fachmann bekannt. In das konditionierende Medium werden empfängerspezifische Zellen eingebracht, entweder kontinuierlich oder in mehreren Schüben.

Unter empfängerspezifischen Zellen werden für den Empfänger autologe oder immunologisch kompatible bzw. verträgliche Zellen verstanden. Es können auch zu unterschiedlichen Besiedlungs- bzw. Behandlungszeitpunkten verschiedene Arten von Zellen aufgegeben werden, so dass sich auf dem Gewebe unterschiedliche Zellschichten aus verschiedenartigen Zellen aufbauen können. Ferner können dem Gewebe Mischungen unterschiedlicher Zellen angeboten werden. Weiterhin können örtlich verschiedene Zellen aufgebracht werden, beispielsweise auf der Oberseite und der Unterseite eines Hauttransplantats unterschiedliche Zellen oder auf der Innenseite und der Außenseite eines röhrenförmigen Gefäßes unterschiedliche Zellen.

Als empfängerverträgliche Zellen kommen grundsätzlich alle Körperzellen, beispielsweise - je nach dem unterliegenden Substrat - auch die nachfolgend beschriebenen in Frage:

Bindegewebszellen (u.a.Fibroblasten, Fibrozyten), Muskelzellen (Myozyten), Endothelzellen, Hautzellen (u.a. Keratinozyten), zu Organzellen differenzierte Zellen (Herzzellen, Nierenzellen, usw.), vorzugsweise bei strukturierten Organen mit Kollagengerüst, allgemein alle Zellen, die für den Umbau eines bestimmten, für die Implantation vorgesehenen Gewebes sinnvollerweise angeboten werden können. Geeignet sind auch die Vorläuferzellen, bevorzugt aus autologen Stammzellen des Empfängers. Zu den Stammzellen gehören die oben bereits genannten.

Bei dem umzubildenden Gewebe bzw. dem Transplantat, das zunächst in nativem Zustand, wie entnommen, vorliegt und dann im Laufe des Verfahrens umgebildet wird, kann es sich grundsätzlich um jedes transplantierbare Gewebe handeln. Insbesondere zählen hierzu: allgemein Gefäße, Aorten, Venen, Aortenklappen, Herzklappen, Organteile und ganze Organe, Hautstücke, Sehnen, Cornea, Knorpel, Knochen, Larynx, Herz, Trachea, Nerven, Miniskus, Diskus intervertebralis, Ureteren, Urethra, Blase, Innenohrknöchelchen, Ohr- und Nasenknorpel, Gelenkknorpel, Bindegewebe, Fettgewebe, Drüsengewebe, Nerven, Muskeln u.a.m.

Für den Umbau des Gewebes mit Hilfe von empfängerverträglichen Zellen werden jeweils Zellen oder Gemische von Zellen ausgewählt, die zu dem jeweiligen Gewebetyp passen. Die empfängerverträglichen, vorzugsweise autologen oder genetisch modifizierten und hierdurch empfängerspezifisch gemachten allogenen oder xenogenen Zellen umfassen neben den erfindungswesentlichen Fibroblasten oder Bindegewebszellen solche Zellen, die für den Aufbau des gewünschten Gewebes geeignet sind, und wahlweise zusätzlich solche, die die Gewebeumbildung mit stimulieren und/oder kontrolieren können, wie z.B. zelluläre Faktoren produzierende Zellen und/oder chemotaktisch beeinflussende Zellen, hierunter vor allem Zellen aus der Familie der Leukozyten (Lymphozyten, Thrombozyten, Makrophagen, Mastzellen, Granulozyten, , so z.B. alle Formen der weißen Blutkörperchen, Granulozyten, Lymphozyten, Makrophagen, Monozyten, Knochenmarkszellen, Milzzellen, Memory-Zellen, Thymuszellen sowie periphere oder zentrale Stammzellen (aus Blut und Knochenmark) oder Stammzellen aus Fettgewebe, vorzugsweise pluripotente Stammzellen.

Bei Herzklappen werden bevorzugt Fibroblasten oder Myofibroblasten, Muskelzellen und/oder Endothelzellen eingesetzt, bei Hauttransplantaten Keratinozyten, Zellen mesodermalen Ursprungs (Mesodermalzellen) und ggf. Hautanhangsgebilde.

Ein wichtiger Aspekt der Erfindung besteht darin, dass die idealerweise autologen Fibroblasten durch die Signalwirkung der initial verbliebenen aber in vitro absterbenden DonorZellen von einem ruhenden in einen aktivierten Phänotypus mutieren können. Dies hat erhebliche Konsequenzen für die Genexpression der empfängerspezifischen bzw. -verträglichen Zellen, die ja auch in einem ruhenden Phänotypus aus gesundem Gewebe gewonnen werden. In vitro wird dann ein "Krankheitszustand" und damit nachfolgend ein "Heilungszustand" induziert. Hierbei kann die Kooperation mit idealerweise empfängereigenen oder empfängerspezifischen Helferzellen verstärkend und permissiv wirken. Die empfängerverträglichen Zellen, die für den Gewebeumbau eingesetzt werden, umfassen daher vorzugsweise auch Makrophagen, aber auch Blutplättchen sowie immunkompetente Zellen wie Lymphozyten.

Zentral für die Erfindung ist, dass die Behandlung fortgesetzt wird bis ein weitgehender, wenn nicht praktisch vollständiger Umbau erreicht oder soweit initiiert wurde, damit eine Weiterführung des ständigen Umbaus in vivo angebahnt ist.

Ein wesentlicher Vorteil der Erfindung ergibt sich daraus, dass Implantationen rascher erfolgen können. Bei der herkömmlichen Methode wurden die Fremdzellen zunächst abgeführt. Dabei wurde die Matrix mechanisch erheblich geschwächt. Anschließend wurde wiederbesiedelt, was einen Zeitraum von wenigstens 24 bis 96 Stunden in Anspruch nahm. Die Stabilität der Matrix nahm während der Wiederbesiedlung allmählich, jedoch abschließend nur bis auf ca. 70 - 80 % des Ausgangswertes (z.B. gemessen an Zugbelastung) zu. Das erfindungsgemäße Verfahren ermöglicht einen Gewebeumbau binnen ca. 4 Tagen (3 bis 6 Tagen), wobei die mechanische Stabilität über den gesamten Zeitraum etwa unverändert bleibt.. Da das Gewebe bereits initial einer physiologischen Stabilität und Belastbarkeit entspricht, verringert sich die Gefahr von Rupturen in der ersten Zeit nach Implantation erheblich. Der Umbau wird im Körper in vivo (nach dem Implantation) fortgesetzt.

Vor der Behandlung mit den empfängerverträglichen Zellen sollte das für die Transplantation vorgesehene autologe, allogene oder xenoge, in nativer Form vorliegende Gewebe sterilisiert werden. Dies hat insbesondere bei xenogenen Geweben zu geschehen, da sicher ausgeschlossen werden sollte, dass artfremde Viren und Bakterien in das frisch erzeugte bioartifizielle Transplantat mit eingetragen werden. Auch bei allogenen Ausgangsgeweben sollte eine Krankheitsübertragung sicher ausgeschlossen werden. Nur ausnahmsweise dürfte es möglich sein, autologe Gewebe für andere Einsatzzwecke umzubilden. Auch hier ist eine Sterilisation sinnvoll, die jedoch weniger aufwendig sein müßte.

Unter einem Gewebe in nativer Form wird ein solches Gewebe verstanden, das im wesentlichen so belassen wurde, wie es entnommen worden ist. Nativ bedeutet in diesem Zusammenhang natürlich, unverändert, nicht-denaturiert. Das Gewebe soll bei Eintritt in die Behandlungsphase mit den empfängervertäglichen Zellen, noch seine nativen Zellen tragen, damit der endogene Stimulus für den Umbau des Gewebes genutzt werden kann. Diese Zellen befinden sich jedoch, wie oben bereits erwähnt, wegen des für Präparation und ggf. Transport verstrichenen Zeitraums im allgemeinen bereits im Zustand des beginnenden Absterbens.

Die Sterilisierung soll möglichst schonend erfolgen. Zum Zwekke der Sterilisierung kann beispielsweise mit einer sterilisierenden Lösung gespült werden oder es kann mit einem Gas sterilisiert werden (Begasung).

Als besonders geeignet wird derzeit eine Sterilisation durch Plasmaionisation angesehen, bei der eine Gasentladung in Anwesenheit von H₂O₂ erfolgt. Hierzu wird eine wässrige Lösung von Wasserstoffperoxid in eine Sterilisationskammer injiziert und vaporisiert. Unter reduziertem Umgebungsdruck wird mit Hilfe einer Radiofrequenzenergie ein niedrig-Temperatur-Plasma appliziert. Hierdurch wird ein elektrisches Feld generiert, welches ein Plasma erzeugt. In dem Plasma-Zustand wird das Wasserstoffperoxid unter Radikalbildung gespalten. Die Radikale stellen die aktive Spezies für die Sterilisation dar. Dieses Verfahren hiterlässt keine toxischen Rückstände, da nach Abschluss der Reaktion die Radikale zu Wasser, Sauerstoff und anderen nicht toxischen Produkten abreagieren. Die Verwendung von Peroxiden entspricht auch einem in vielen Zellen vorkommenden natürlichen Prozess (z.B. in Makrophagen).

Gegebenenfalls kann der Erfolg der Sterilisierung spezifisch geprüft werden, in dem beispielsweise nach der Sterilisierung auf Anwesenheit bestimmter Viren oder Bakterien, die strikt unterbunden werden sollen, getestet wird.

Das für die Transplantation vorgesehene Gewebe kann nach seiner Präparation vor einer möglicherweise erforderlichen Sterilisierung zusätzlichen nicht-denaturierenden Verfahrensschritten, z.B. einem Spülen ausgesetzt werden. Auch ein schonendes Einfrieren des nativen Transplantat-Gewebes ist möglich, sofern hierbei tiefgreifendere Gewebeänderungen vermieden werden.

In Weiterbildung der Erfindung ist vorgesehen, dass dem konditionierenden Medium, während der Behandlung mit empfängerverträglichen Zellen oder danach, zusätzlich zelluläre Mediatoren und/oder Faktoren oder chemische Mediatoren zugesetzt werden. Die Wirkung bestimmter Faktoren wurde bereits untersucht, so dass der Fachmann gezielt Zellwachstumsfaktoren, zelldifferenzierende Faktoren, chemotaktische Faktoren und andere auswählen und einsetzen kann. Insbesondere können verwendet werden:
Neuropeptide: Diese können die Fähigkeit besitzen, mesenchymale Zellen zu aktivieren. Bei Fibroblasten kann die Proliferation und Chemotaxis beeinflusst werden. Unter den geeigneten Neuropeptiden sind insbesondere zu nennen: Neurokinin (Neurokinin A (NKA)), Substanz P (SP), vasoactives Intestinal-Peptid (VIP), Calcitonin-Gen-bezogenes Peptid (calcitonin-generelated peptide (CGRP));
als weitere vorwiegend chemotaktisch und/oder proliferationssteuernd wirkende Mediatoren/Faktoren können - je nach Zellund Gewebetypus - verwendet werden:
   Fibronectin (Fn), Cytokine, wie Interleukin-1-beta (IL-1 beta), Interleukin-6 (IL-6), Interleukin-8 (IL-8), Interferone, wie Interferon-gamma (IFN-gamma), Granulozyten-Makrophagenkolonie-stimulierender Faktor (granulocytemacrophage colony stimulation factor (GM-CSF)), Transformations-Wachstumsfaktor beta 1 (transforming growth factor - beta 1 (TGF-beta 1)), osteogenes Protein-1 (osteogenic protein-1 (OP-1)), recombinantes humanes osteogenes Protein-1 (rhOP-1), Plasminogenaktivator vom Urokinasetyp (urokinase-type plasminogen activator (u-PA)), PDGF (platelet-derived growth factor), Insbesondere PDGF AA, PDGF AB, PDGF BB, HGF (hepatocyte growth factor), VEGF (vascular endothelial growth factor), FGF (fibroblast growth factor), ECGF (endothelial cell growth factor), Glycoproteine, wie alpha-2-Macroglobulin (alpha-2M), Clara Zellprotein (CC-16), Platelet factor 4, beta-Thromboglobulin, Neutrophile-aktivierendes Paptid-1, ferner auch synthetische Mediatoren, wie z.B. Mannose-6-phosphat, Adaptil und andere.

Die konkrete Funktion der einzelnen Mediatoren, Faktoren, Co-Faktoren ist dem Fachmann auf dem Gebiet für isolierte Zellen bekannt, so dass er im Rahmen der hier beschriebenen Erfindung für den jeweiligen Zweck geeignete Mediatoren/Faktoren auswählen kann.

In Weiterbildung der Erfindung ist vorgesehen, daß das Verfahren so geführt wird, daß dem konditionierenden Medium und/oder Gewebe immunkompetente Zellen, insbesondere Makrophagen, die zelluläre Mediatoren und/oder Faktoren an das konditionierende Medium abgeben, zugeführt werden. Insbesondere kann das konditionierende Medium hierfür aus autologen, d.h. empfängereigenem Blut bestehen, hiermit zeitweise angereichert oder durch Blut zeitweise ersetzt werden. Hierdurch können Makrophagen aus dem Blut selektiv an dem Gewebe adhärieren. Die Makrophagen erhalten immunstimulatorische Reize aus dem noch nicht oder unvollständig umgebildeten Gewebe und setzen hierdurch zelltypspezifische Mediatoren frei, die den Umbau beschleunigen. Blutplättchen lysieren und setzen beispielsweise Wachstumsfaktoren frei. Der Gewebeumbau wird angeregt, gesteuert und beschleunigt.

In weiterer Fortbildung der Erfindung ist vorgesehen, das verfahren so zu führen, dass aus einer Kultur immunkompetenter Zellen, inssbesondere einer Makrophagen-Kultur, zelluläre Mediatoren und/oder Faktoren an das konditionierende Medium abgegeben oder in dieses überführt werden. Diese Kultur kann auch Lymphozyten oder Blutplättchen enthalten. Weiterhin können hier auch Stammzellen zugesetzt sein.

Die Kultur zur verstärkten Abgabe von Faktoren oder Mediatoren geeigneter, beispielsweise immunkompetenter Zellen oder die Makrophagen-Kultur kann in einem Bioreaktor erfolgen, der mit dem Reaktor, in dem das bioartifzielle Transplantat vorbereitet und behandelt wird, in geeigneter Weise verbunden ist. Aus dem Bioreaktor abgenommene Faktoren können dem umlaufenden oder schubweise zugeführten konditionierenden Kulturmedium in geeigneter Menge zugeführt werden.

Durch einen geeigneten Bioreaktor kann hier ein druck- und belastungsabhängiges Remodelling, z.B. durch pulsatilen Perfusionsbetrieb, beispielsweise bei Gefäßen und Herzklappen, durchgeführt werden, das sich sehr positiv auf die Naturähnlichkeit des umgebildeten Gewebes auswirkt. Es verbessert die der normalen Physiologie entsprechende Ausprägung des bioartifiziellen Gewebes in vitro.

Alternativ kann die Makrophagen-Kultur, bzw. die Kultur sonstiger immunkompetenter Zellen, während der Schritte der Behandlung mit empfängerverträglichen Zellen durch ein für die zellulären Mediatoren und/oder Faktoren durchlässige Folie, Membran oder Trennwand von dem konditionierenden Medium getrennt gehalten werden und die gebildeten Mediatoren und/oder Faktoren können kontinuierlich in das konditionierende Medium abgegeben werden.

Die Behandlung des für die Transplantation vorgesehenen Gewebes wird im allgemeinen in einem Bioreaktor erfolgen, in dem das Kulturmedium innerhalb eines bestimmten Raumes vorgehalten und ggf. umgewälzt wird. Innerhalb dieses Raumes kann mit einer permeablen Trennwand ein Kulturraum für die Kultur der immunkompetenten Zellen oder Makrophagen ausgebildet werden, so dass die gebildeten zellulären Mediatoren und/oder Faktoren ständig in das konditionierende Medium auswandern können. Alternativ können die immunkompetenten Zellen auch gesondert kultiviert und die Zellkulturprodukte können dem Bioreaktor, der zur Gewebekultur verwendet wird, zugeführt werden. Zusätzlich kann das Produkt (d.h., das Organ oder allgemein das Gewebe) zu Konditionierungszwecken mit oder unter Zusatz von empfängerspezifischem Vollblut oder einzelner Blutkomponenten (Proteine, Fibronektin, Thrombin, Fibrinogen, Plasma, Serum, zelluläre Bestandteile) perfundiert bzw. koinkubiert werden. Als immunkompetente Zellen können insbesondere verwendet werden:
Alle Formen der weißen Blutkörperchen, Granulozyten, Lymphozyten, Makrophagen, Monozyten, Knochenmarkszellen, Milzzellen, Memory-Zellen, Thymuszellen
Beide vorgenannten Alternativen können auch kombiniert werden, indem sowohl immunkompetente, bzw. immunmodulatorische Zellen innerhalb oder außerhalb des Gewebe-Bioreaktors cokultiviert werden, um bestimmte Mediatoren/Faktoren zu erzeugen, und
gleichzeitig auch noch natürlich gewonnene oder synthetische Mediatoren/Faktoren dem Gewebekulturmedium zugesetzt werden.

Die Co-Kultur immunkompetenter Zellen, die Mediatoren, Faktoren, Co-Faktoren produzieren und an das konditionierende Medium abgeben, ist besonders vorteilhaft, da für den jeweiligen Zweck besonders geeignete Mediatoren/Faktoren während eines ohnehin erforderlichen Kulturschritts mitproduziert werden können, so dass auf die Verwendung zusätzlicher teurer und weniger spezifischer Faktoren verzichtet werden kann.

Im folgenden wird die Erfindung anhand einiger Beispiele beschrieben:

### Beispiel 1

### Transdifferenzierung einer allogenen kryokonservierten Vene in eine autologe Arterie:

Kryokonservierte allogene Venen werden ohne weitere Behandlung steril in einen Bioreaktor eingebracht und mit idealerweise serumfreien oder autologem Serum / Plasma angreichertem Medium perfundiert. Vorexpandierte autologe aus einer Arterie stammende Fibroblasten und glatte Muskelzellen werden an der Außenseite der vormals kryokonservierten Vene angebracht. Dies geschieht hier durch Aufbringen in einem (autologen) Fibringel, Kollagengel, in synthetischen Kleberproteinen aus Muscheln durch Auftropfen oder Aufstreichen der mit dem Klebervermischten Zellen vor der Kultivierung oder durch Auftropfen oder Aufstreichen einer Zellsuspension in Medium. Innerhalb des Gefäßlumens werden Endothelzellen (fakultativ nach einer Vorbesiedlung mit Myofibroblasten) aufgebracht. Dies geschieht unter langsamen Rotieren des Gefäßes innerhalb eines Bioreaktors, wobei ein Bioreaktor jede hierfür geeignete Vorrichtung sein kann. Die Fibroblasten werden durch den Zelldetritus der abgestorbenen Zellen angeregt neue Matrix zu synthetisieren, neue Gewebestrukturen aufzubauen und sich verstärkt in das Gewebe/die Matrix zu integrieren. Ein mehrlagiger Muskelzellmantel bildet sich innerhalb weniger Tage aus.

Das arterialisierte (umgebildete) Gefäß ist somit ohne Stabilitätsverlust (wie überlicherweise nach der Azellularisierung bis zu < 20 % initiale Stärke), sehr rasch transplantationsbereit.

### Beispiel 2

### Transdifferenzierung einer xenogenen kryokonservierten Arterie in eine autologe humane Arterie:

Xenogene Arterien werden ohne jegliche Azellularisierung mit autologen arteriellen Gefäßzellen in Analogie zu dem ersten Beispiel, jedoch noch ohne Endothelzellen besiedelt. Das chimäre Konstrukt (umgebildetes Gewebe) wird mit autologem Blut durchspült. In dieser Phase adhärieren Makrophagen selektiv an der exponierten Matrix. Lymphozyten erhalten immunstimulatorische Reize durch die xenogene Matrix. Bluttplättchen lysieren und setzen Wachstumsfaktoren wie PDGF frei. Nach einer Einwirkzeit von ca. 4 Std. (ausreichend für die Makrophagenadhäsion) wird das autologe Blut wieder mit plasmaangereichertem Kulturmedium ersetzt und für mehrere Tage (ca. 3-10) nachkultiviert. In dieser Phase kommt es zu einem akzelerierten Matrixturnover durch die für die Besiedlung zugeführten (autologen) Myofibroblasten. Durch pulsatile Belastungen erfolgt eine gerichtete druckgesteuerte Deposition neuer Matrixmolekule und Fasern. Ebenso wird die orientierte Integration der neuformierten Zellverbände ermöglicht.

Alternativ können Präparationen von Blutplättchen (Gewinnung bei ca. 3000 g und weiße Blutkörperchen (1800 g) separat in unterschiedlichen Bereichen des Bioreaktors oder synchron in einer gesonderten Apparatur kokultiviert werden. In letzterem Falle werden die so erhaltenen Kulturprodukte der Gewebekultur in dem eigentlichen Gewebe-Bioreaktor zugeführt.

## Patentansprüche

1. Verfahren zur Herstellung eines bioartifiziellen Transplantates aus einem für die Transplantation vorgesehenen biologischen Gewebe und darauf aufgebrachten empfängerverträglichen Zellen, **dadurch gekennzeichnet,**
- **dass** dem für die Transplantation vorgesehenen autologen, allogenen oder xenogenen, in nativer Form (nicht azellulerisiert) vorliegenden und nicht mit exogenen Wachstumsfaktoren vorbehandelten Gewebe in einem konditionierenden Medium empfängerverträgliche Zellen zugeführt werden, die wenigstens ausgewählte zu einem Remodelling der Trägerstrukturen befähigte Zellen umfassen,
- **dass** die Behandlung des Transplantates fortgesetzt wird bis eine weitgehende Umbildung des usprünglichen nativen Gewebes in ein im wesentlichen die zugeführten empfänger verträglichen Zellen aufweisendes Gewebe erzielt worden ist.

2. Verfahren nach Anspruch 1, **dadurch gekennzeichnet, dass** die zum Remodelling der Trägerstrukturen befähigten Zellen Bindegewebszellen oder deren Vorläufer sind, insbesondere Fibroblasten oder Bindegewebs-Vorläuferzellen, vorzugsweise aus autologen Stammzellen.

3. Verfahren nach Anspruch 1, **dadurch gekennzeichnet, dass** das umzubildende Gewebe ein Herzgewebe ist und die zum Remodelling befähigten Zellen glatte Muskelzellen sind.

4. Verfahren nach Anspruch 1, **dadurch gekennzeichnet, dass** die zum Remodelling befähigten Zellen Makrophagen sind.

5. Verfahren nach einem der Ansprüche 1 bis 4, **dadurch gekennzeichnet, dass** die empfängerverträglichen Zellen einmalig zu Beginn der Kultur, mehrfach in Intervallen oder kontinuierlich innerhalb des Mediums zugeführt werden.

6. Verfahren nach einem der Ansprüche 1 bis 5, **dadurch gekennzeichnet, dass** die empfängerverträglichen Zellen auf das umzubildende native Gewebe aufgetropft oder aufgestrichen, oder kontinuierlich oder schubweise mit dem konditionierenden Medium zugeführt werden.

7. Verfahren nach einem der Ansprüche 1 bis 6, **dadurch gekennzeichnet, dass** die zuzuführenden empfängerverträglichen Zellen vermischt mit einem biologisch verträglichen Kleber, der insbesondere Fibrin, Kollagen oder Kleberproteinen enthalten kann, oder in einer Kulturmedium-Suspension aufgegeben werden.

8. Verfahren nach einem der Ansprüche 1 bis 7, **dadurch gekennzeichnet, dass** die Behandlung des Transplantats in der Kultur unter mehrmaligem Austausch oder bei kontinuierlichem Durchlauf des Mediums durchgeführt wird.

9. Verfahren nach einem der Ansprüche 1 bis 8, **dadurch gekennzeichnet, dass** dem konditionierenden Medium während der Behandlung mit empfängerverträglichen Zellen zelluläre Mediatoren und/oder Faktoren oder chemische Mediatoren zugesetzt werden.

10. Verfahren nach einem der Ansprüche 1 bis 9, **dadurch gekennzeichnet, dass** das Verfahren so geführt wird, dass dem konditionierenden Medium und/oder dem Gewebe zur Abgabe zellulärer Mediatoren und/oder Faktoren besonders befähigte und aktivierbare Zellen, insbesondere Makrophagen, zusätzlich zugeführt werden.

11. Verfahren nach Anspruch 10, **dadurch gekennzeichnet, dass** die zur Abgabe zellulärer Mediatoren und/oder Faktoren besonders befähigten und aktivierbaren Zellen, insbesondere die Makrophagen, in einer Kultur vorgehalten werden, vorzugsweise in einem Bioreaktor, weiter vorzugsweise in demselben Bioreaktor, in dem auch das Transplantat behandelt wird.

12. Verfahren nach Anspruch 11, **dadurch gekennzeichnet, dass** die Makrophagen- oder entsprechende Zellkultur während der Besiedlung oder Behandlung mit empfängerverträglichen Zellen durch eine für die zellulären Mediatoren und/oder Faktoren durchlässige Folie, Membran oder Trennwand von dem konditionierenden Medium getrennt gehalten wird und die gebildeten Mediatoren und/oder Faktoren kontinuierlich in das konditionierende Medium abgegeben werden.

13. Verfahren nach einem der Ansprüche 1 bis 12, **dadurch gekennzeichnet, dass** das für die Transplantation vorgesehene autologe, allogene oder xenogene, in nativer Form vorliegende Gewebe zunächst sterilisiert wird, vorzugsweise durch Spülen mit einer sterilen Lösung oder durch Begasen.

14. Verfahren nach Anspruch 13, **dadurch gekennzeichnet, dass** die Sterilisation durch eine Plasmaionisation mit H₂O₂ erfolgt.

15. Verfahren nach einem der Ansprüche 1 bis 14, **dadurch gekennzeichnet, dass** das für die Transplantation vorgesehene Gewebe nach seiner Präparation, vorzugsweise vor oder nach der Sterilisierung, zusätzlichen nicht-denaturierenden Verfahrensschritten ausgesetzt wird.

16. Verfahren nach einem der Ansprüche 1 bis 15, **dadurch gekennzeichnet, dass** das für die Transplantation vorgesehene Gewebe nach seiner Präparation, vorzugsweise vor oder nach der Sterilisierung, zusätzlich ein- oder mehrmals gespült wird.

17. Verfahren nach einem der Ansprüche 1 bis 16, **dadurch gekennzeichnet, dass** die empfängerverträglichen Zellen autologe Zellen des Transplantat-Empfängers sind.

18. Verfahren nach einem der Ansprüche 1 bis 16, **dadurch gekennzeichnet, dass** die empfängerverträglichen Zellen für den Empfänger als verträglich ausgewählte allogene oder genetisch veränderte allogene Zellen sind.

## Claims

1. Method for the manufacture of a bioartifical transplant from a biological tissue intended for transplantation and recipient-compatible cells attached to such tissue,
**characterized in**
- **that** recipient-compatible cells comprising at least selected cells capable of remodelling the carrier structures are added in a conditioning medium to the autologous, allogenic or xenogenic tissue intended for transplantation, which is present in a native, non-acellularized form and has not been pre-treated with exogenous growth factors,
- **that** the treatment of the transplant is continued until achieving large transformation of the initially native tissue into a tissue essentially containing the added recipient-compatible cells.

2. Method according to Claim 1, **characterized in that** the cells capable or remodelling the carrier structures are connective tissue cells or their precursors, especially fibroblasts or connective tissue precursors, preferably from autologous stem cells.

3. Method according to Claim 1, **characterized in that** the tissue to be transformed is a heart tissue and that the cells capable of remodelling are smooth muscle cells.

4. Method according to Claim 1, **characterized in that** the cells capable of remodelling are macrophages.

5. Method according to any of the Claims 1 to 4, **characterized in that** the recipient-compatible cells are added only at the beginning of the culture, at repeated intervals, or continuously within the medium.

6. Method according to any of the Claims 1 to 5, **characterized in that** the recipient-compatible cells are applied by dropping or spreading onto the native tissue to be transformed, or supplied continuously or in batches with the conditioning medium.

7. Method according to any of the Claims 1 to 6, **characterized in that** the recipient-compatible cells to be added are supplied mixed with a biologically compatible cement, which may contain especially fibrin, collagen, or gluten, or in a culture medium suspension.

8. Method according to any of the Claims 1 to 7, **characterized in that** the in-culture treatment of the transplant is performed with repeated replacement or continuous flow of the medium.

9. Method according to any of the Claims 1 to 8, **characterized in that** cellular mediators and/or factors, or chemical mediators are added to the conditioning medium during the treatment with recipient-compatible cells.

10. Method according to any of the Claims 1 to 9, **characterized in that** the method is controlled in a way to additionally supply the conditioning medium and/or the tissue with cells specially activatable and capable of delivering cellular mediators and/or factors, especially macrophages.

11. Method according to Claim 10, **characterized in that** the cells specially activatable and capable of delivering cellular mediators and/or factors, especially the macrophages, are placed ready in a culture, preferably in a bioreactor, further preferably in the same bioreactor as used for treating the transplant.

12. Method according to Claim 11, **characterized in that** the macrophage or respective cell culture is kept apart from the conditioning medium during the seeding or treatment with recipient-compatible cells by a film, diaphragm or partition permeable to the cellular mediators and/or factors and that the mediators and/or factors formed are continuously fed into the conditioning medium.

13. Method according to any of the Claims 1 to 12, **characterized in that** the autologous, allogenic or xenogenic tissue intended for the transplantation, which is present in a native form, is first of all sterilized, preferably by washing in a sterile solution or by gassing.

14. Method according to Claim 13, **characterized in that** sterilization is by plasma ionization with H₂O₂.

15. Method according to any of the Claims 1 to 14, **characterized in that** the tissue intended for transplantation is subjected to additional non-denaturing process steps following its preparation, preferably before or after its sterilization.

16. Method according to any of the Claims 1 to 15, **characterized in that** the tissue intended for transplantation undergoes one or several additional washes following its preparation, preferably before or after sterilization.

17. Method according to any of the Claims 1 to 16, **characterized in that** the recipient-compatible cells are autologous cells of the transplant recipient.

18. Method according to any of the Claims 1 to 16, **characterized in that** the recipient-compatible cells are allogenic cells selected as compatible or genetically modified for the recipient.

## Revendications

1. Procédé pour la fabrication d'un transplant bioartificiel à partir d'un tissu biologique prévu pour la transplantation et de cellules y apposées compatibles avec le receveur,
**caractérisé en ce**
- **que** des cellules compatibles avec le receveur et contenant des cellules, au moins sélectionnées, aptes à remodeler les structures de support sont, dans un milieu conditionnant, apportées au tissu autologue, allogénique ou xénogénique prévu pour la transplantation, qui se présente sous forme native non acellularisée et n'a pas subi de traitement antérieur par des facteurs de croissance exogènes,
- **que** le traitement du transplant continue jusqu'à ce qu'une large transformation du tissu initialement natif en un tissu contenant l'essentiel des cellules compatibles avec le receveur rajoutées soit atteinte.

2. Procédé selon la revendication 1, **caractérisé en ce que** les cellules aptes au remodelage des structures de support sont des cellules de tissu conjonctif ou leurs précurseurs, notamment des fibroblastes ou des cellules précurseurs de tissu conjonctif, de préférence en provenance de cellules souches.

3. Procédé selon la revendication 1, **caractérisé en ce que** le tissu à transformer est un tissu cardiaque et que les cellules aptes au remodelage sont des cellules musculaires lisses.

4. Procédé selon la revendication 1, **caractérisé en ce que** les cellules aptes au remodelage sont des macrophages.

5. Procédé selon l'une des revendications 1 à 4, **caractérisé en ce que** les cellules compatibles avec le receveur sont introduites une seule fois au début de la culture, à plusieurs reprises à des intervalles donnés ou en continu dans le milieu.

6. Procédé selon l'une des revendications 1 à 5, **caractérisé en ce que** les cellules compatibles avec le receveur sont appliquées par dégouttement ou par enduction sur le tissu natif à transformer ou apportées en continu ou en discontinu avec le milieu conditionnant.

7. Procédé selon l'une des revendications 1 à 6, **caractérisé en ce que** les cellules compatibles avec le receveur à apporter sont apportées mélangées avec un liant biologiquement compatible pouvant contenir notamment de la fibrine, du collagène ou du gluten, ou dans une suspension de milieu de culture.

8. Procédé selon l'une des revendications 1 à 7, **caractérisé en ce que** le traitement du transplant dans la culture se déroule avec un remplacement répété ou l'écoulement continu du milieu.

9. Procédé selon l'une des revendications 1 à 8, **caractérisé en ce que**, pendant le traitement par les cellules compatibles avec le receveur, des médiateurs et/ou facteurs cellulaires ou des médiateurs chimiques sont apportés au milieu conditionnant.

10. Procédé selon l'une des revendications 1 à 9, **caractérisé en ce que** la conduite du procédé est telle que le milieu conditionnant et/ou le tissu est complémentairement additionné de cellules spécialement activables et aptes à fournir des médiateurs et/ou facteurs cellulaires, notamment de macrophages.

11. Procédé selon la revendication 10, **caractérisé en ce que** les cellules spécialement activables et aptes à fournir des médiateurs et/ou facteurs cellulaires, notamment les macrophages, sont tenues à disposition dans une culture, de préférence dans un bioréacteur et de préférence dans le même bioréacteur utilisé pour le traitement du transplant.

12. Procédé selon la revendication 11, **caractérisé en ce que** la culture de macrophages ou culture correspondante est, pendant l'ensemencement ou le traitement par des cellules compatibles avec le receveur, tenue isolée du milieu conditionnant par un film, une membrane ou un diaphragme perméable aux médiateurs ou facteurs cellulaires et que les médiateurs et/ou facteurs formés sont introduits en continu dans le milieu conditionnant.

13. Procédé selon l'une des revendications 1 à 12, **caractérisé en ce que** le tissu autologue, allogénique ou xénogénique prévu pour la transplantation et se présentant sous forme native est dans un premier pas stérilisé, de préférence par lavage dans une solution stérile ou par gazage.

14. Procédé selon la revendication 13, **caractérisé en ce que** la stérilisation se fait par ionisation par plasma avec H₂O₂.

15. Procédé selon l'une des revendications 1 à 14, **caractérisé en ce que** le tissu prévu pour la transplantation est assujetti à des opérations non dénaturantes supplémentaires après sa préparation, de préférence avant ou après sa stérilisation.

16. Procédé selon l'une des revendications 1 à 15, **caractérisé en ce que** le tissu prévu pour la transplantation fait l'objet d'un ou de plusieurs lavages supplémentaires, de préférence avant ou après la stérilisation.

17. Procédé selon l'une des revendications 1 à 16, **caractérisé en ce que** les cellules compatibles avec le receveur sont des cellules autologues du patient receveur du transplant.

18. Procédé selon l'une des revendications 1 à 16, **caractérisé en ce que** les cellules compatibles avec le receveur sont des cellules allogéniques sélectionnées comme compatibles ou génétiquement modifiées pour le patient receveur.
